# EUROPEAN PATENT APPLICATION

(11) **EP 1 607 381 A1**
(43) Date of publication of application: **21.12.2005**
(21) Application number: 04722722.8
(22) Date of filing: 23.03.2004
(51) Int. Cl.: C07C 41/54, C07C 43/315, C07D 231/12, C07D 239/42

(54) **PROCESS FOR PRODUCING TETRAALKOXYPROPANE AND DERIVATIVE THEREOF**

(30) Priority: 26.03.2003 JP 2003085490; 26.03.2003 JP 2003085489; 26.03.2003 JP 2003085491
(71) Applicant: NIPPON CARBIDE KOGYO KABUSHIKI KAISHA, Tokyo 108-8466 (JP)
(72) Inventor: MITA, Shinya, 9300103 (JP); KAKINUMA, Shinichi, 9370801 (JP); MUROTANI, Masahiro, 9300901 (JP)
(74) Representative: Becker Kurig Straus
(86) International application number: PCT/JP2004/003908
(87) International publication number: WO 2004/085358

(57) **Abstract**

In view of the fact that a methyl vinyl ether, which is useful as a starting material for the synthesis of tetraalkoxypropane useful as a skeleton-forming agent having a high reactivity usable as a starting material, etc. for a pharmaceutical and agrochemical intermediates such as a pyrazole derivative or pyrimidine derivative, particularly 1,1,3,3-tetramethoxypropane, is in a gaseous state and difficult to use for industrial production, tetraalkoxypropane useful as a skeleton-forming agent for a pharmaceutical and agrochemical intermediate such as a pyrazole derivative or pyrimidine derivative is easily produced on an industrial scale by using the industrially useable propoxyvinyl ether, as a starting material, without using a gaseous state methyl vinyl ether.

## Description

### TECHNICAL FIELD

The present invention relates to a method for producing a tetraalkoxypropane useful as a skeleton-forming agent having a high reactivity capable of using as a starting material for a pharmaceutical and agrochemical intermediates such as a pyrazole derivative or pyrimidine derivative and a method for producing a tetraalkoxypropane derivative such as a pyrazole derivative, pyrimidine derivative, etc. using the same.

### BACKGROUND ART

In the past, 1,1,3,3-tetraalkoxypropane has been known. For example, Japanese Unexamined Patent Publication (Kokai) No. 57-158735 (1982) and *Yakugaku Zasshi* (*Journal of the Pharmaceutical Society of Japan*) 82, 1962, pp. 269 to 273, describe methods for producing 1,1,3,3-tetramethoxypropane and 1,1,3,3-tetraethoxypropane. According to these publications, the desired 1,1,3,3-tetramethoxy(or ethoxy)propane, etc. is synthesized from about 3 moles of an orthoformic acid ester and 1 mole of a vinyl ether corresponding to that ester.

The method for synthesizing, from a symmetric or asymmetric tetraalkoxypropane, a pyrazole derivative or pyrimidine derivative and tetraalkoxypropane derivative as a skeleton-forming agent are known. For example, Japanese Examined Patent Publication (Kokoku) No. 28-3825 and Japanese Examined Patent Publication (Kokoku) No. 29-8421 disclose methods of synthesis of a pyrimidine derivative such as 2-aminopyrimidine. Further, U.S. Patent No. 3,925,552 describes a method of synthesis of a pyrazole derivative such as 1-carboxylamidinopyrazole. According to these publications, the desired pyrimidine derivative or pyrazole derivative is obtained by the conversion from 1,1,3,3-tetramethoxy-propane or 1,1,3,3-tetraethoxypropane.

However, for example, for the synthesis of 1,1,3,3-tetramethoxypropane methyl vinyl ether should be used as the vinyl ether corresponding to methyl orthoformate, but methyl vinyl ether is in a gaseous state, and therefor, while laboratory synthesis of 1,1,3,3-tetramethoxypropane is possible, use of industrially large amounts of methyl vinyl ether etc. has been difficult.

Further, for the synthesis of 1,1,3,3-tetraethoxypropane, ethyl vinyl ether corresponding to ethyl orthoformate is used, but the ethyl vinyl ether has a boiling point of 36 to 37°C and is, therefore, especially combustible. Use in large quantities has been industrially difficult in the same way as methyl vinyl ether.

Further, for 1,1,3,3-tetramethoxypropane and 1,1,3,3-tetraethoxypropane useful as a skeleton-forming agent, for example, as described in Japanese Unexamined Patent Publication (Kokai) No. 57-158735, an alkyl orthoformate and the corresponding vinyl ether thereof are used in the synthesis process thereof. Further, for example, as shown in Japanese Unexamined Patent Publication (Kokai) No. 58-96037, they are produced from alkyl formate, oxirane and a vinyl ether corresponding to the alkyl formate.

Specifically, for the synthesize of 1,1,3,3-tetramethoxy-propane, methyl orthoformate and the corresponding methyl vinyl ether thereof are used, while to synthesize 1,1,3,3-tetraethoxypropane, ethyl orthoformate and the corresponding ethyl vinyl ether thereof are used. Further, for the production of 1,3,3-triethoxy-1-methylether or 1,3,3-trimethoxy-1-ethyl ether useful as a skeleton-forming agent, similarly the use of methyl vinyl ether or ethyl vinyl ether is essential.

### DISCLOSURE OF THE INVENTION

The object of the present invention is to industrially produce 1,1,3,3-tetraalkoxypropane using the industrially utilizable vinyl ether, when producing 1,1,3,3-tetraalkoxypropane.

Another object of the present invention is to provide a method for the industrial production of tetraalkoxypropane derivatives such as a pyrazole derivative and pyrimidine derivative, which are pharmaceutical and agrochemical intermediates.

In accordance with a first aspect of the present invention, there is provided a method for producing 1,1,3,3-tetraalkoxypropane having the formula (I): 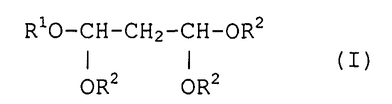 wherein R¹ indicates CH₃, C₂H₅ or C₃H₇ and R² independently indicates CH₃ or C₂H₅, comprising using, as starting materials, an orthoformic acid ester having the formula (II) : 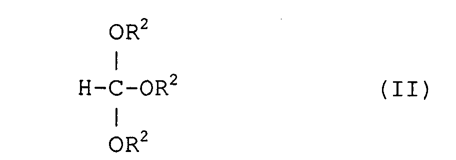 wherein R² is as defined above and
a vinyl ether having the formula (III):

CH₂=CH-OR³ (III)

wherein R³ indicates C₃H₇.

In accordance with a second aspect of the present invention, there is provided a method for producing a tetraalkoxypropane derivative using, as a starting material, a tetraalkoxypropane having the formula (IV): 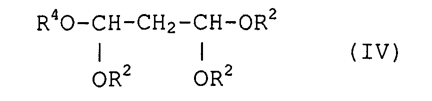 wherein R⁴ indicates CH₃, C₂H₅, C₃H₇ or C₄H₉ and R² independently indicates CH₃ or C₂H₅.

### BEST MODE FOR CARRYING OUT THE INVENTION

Embodiments of the present invention will now be explained in detail.

The inventors engaged in research for the purpose of the synthesis of tetraalkoxypropanes from industrially utilizable vinyl ethers. The tetraalkoxypropanes are highly reactive skeleton-forming agent and useful, as a starting material, for example, for pharmaceutical and agrochemical intermediates such as pyrazole derivatives and pyrimidine derivatives and also have a wide range of applications and, as a result, found that the use of propoxyvinyl ether having a relatively high boiling point eliminates the above disadvantages and enables the novel asymmetric 1,3,3-trimethoxy-1-(n- or iso-)propoxypropane and 1,3,3-triethoxy-1-(n- or iso-)propoxypropane to be produced and that, even if using propoxyvinyl ether, 1,1,3,3-tetramethoxypropane and 1,1,3,3-tetraethoxypropane can be produced, whereby the present invention has been completed. Further, we found that it is possible to use a mixture of these vinyl ethers to easily synthesize a pyrazole derivative or pyrimidine derivative or other tetraalkoxypropane derivative. Thus, the present invention has been completed.

Normally, for example, as the method for producing 1,1,3,3-tetraalkoxypropane having superior properties and extremely useful as a skeleton-forming agent having a high reactivity useful as a material for a pharmaceutical and agrochemical intermediate such as pyrazole or pyrimidine, orthoformic acid ester and its corresponding vinyl ether are used for synthesis. When producing 1,1,3,3-tetramethoxypropane, as the vinyl ether corresponding to methyl orthoformate, gaseous state methyl vinyl ether must be used. Large scale industrial production was therefore difficult.

Further, when producing 1,1,3,3-tetraethoxy-propane, it is necessary to use the specially combustible ethyl vinyl ether as the vinyl ether corresponding to ethyl orthoformate, so again large scale industrial production was difficult.

The inventors were allowed to react propyl vinyl ether with orthoformic acid ester for the purpose of newly synthesizing asymmetric 1,3,3-trialkoxy-1-propoxypropane to obtain the desired substance and succeeded in establishing a method for producing not only the asymmetric form, but also a symmetric form from propoxyvinyl ether, which is a material suitable for the production on an industrial scale.

The novel, asymmetric 1,3,3-trialkoxy-1-propoxypropane may be produced according to the following reaction formula:

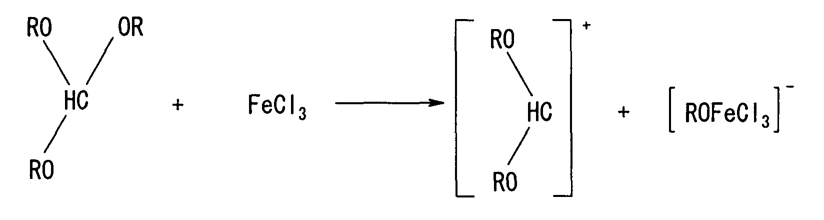

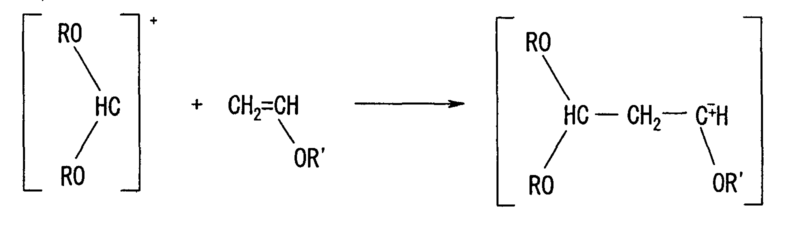

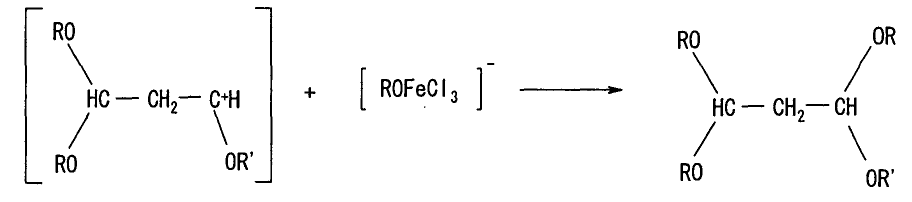

Next, this novel, asymmetric 1,3,3-trialkoxy-1-propoxypropane is believed to be 1,1,3,3-tetraalkoxy-propane by a transalkoxylation reaction with the above product.

Further, the transalkoxylation reaction results in the production of not only the asymmetric form, but also the symmetric tetraalkoxypropane. The ratio of production of the symmetric form and asymmetric form can be freely adjusted by changing the molar ratio of the material propoxyvinyl ether and trialkyl orthoformate and the reaction temperature. For example, in systems using orthoformic acid esters in large excess, the symmetric form is preferentially produced and, from the viewpoint of the yield, a vinyl ether can be effectively utilized. Further, it is possible to collect and recycle the excessive amount of orthoformic acid ester.

As a specific method for the synthesis of the compound according to the present invention, for example, the following method may be mentioned.

For example, a glass flask is charged with propyl vinyl ether and heated. Next, as a reaction catalyst, for example, Lewis acid compound such as anhydrous iron (III) chloride, boron trifluoride is added in an amount of about 0.5 to 2.0 parts by weight, based upon 100 parts by weight of the vinyl ether. While stirring, for example, ortho-ester such as methyl orthoformate is added in an amount of about 1.0 to 1.3 moles, based upon 1 mole of the vinyl ether, and the mixture is reacted at, for example, about -30°C to about 50°C.

After the end of the reaction, the reaction mixture is distilled in vacuo to first obtain the novel compound of the present invention, i.e., 1,3,3-trialkoxy-1-propoxypropane and then 1,1,3,3-tetraalkoxypropane.

Next, according to the present invention, when producing a tetraalkoxypropane derivative, the industrially utilizable propoxyvinyl ether or butoxyvinyl ether is used as the vinyl ether for synthesizing the skeleton-forming agent, tetraalkoxypropane to produce, for example, 1,1,3,3-tetramethoxypropane, 1,1,3,3-tetraethoxypropane, 1,3,3-trimethoxy-1-(propoxy or butoxy)propane, 1,3,3-triethoxy-1-(propoxy or butoxy)propane and the mixtures thereof. These tetraalkoxypropanes are used, as a skeleton-forming agent for the production of tetraalkoxypropane derivatives.

In the present invention, propoxyvinyl ether or butoxyvinyl ether and trialkyl orthoformate are reacted in the presence of a Lewis acid catalyst to form a tetraalkoxypropane. As the orthoformic acid ester, trimethyl orthoformate, triethyl orthoformate, tri(n- or iso-)propyl orthoformate, tri(n- or iso-)butyl orthoformate, etc. may be used, but trimethyl orthoformate is preferably used in view of the ease of acquisition, etc. The amount of use is 1 to 10 moles or so, based upon 1 mole of the vinyl ether.

As the Lewis acid, boron trifluoride, boron trifluoride etherate, aluminum (III) chloride, stannous (II) chloride, hydrogen fluoride, merculic chloride, iron (III) chloride, etc. may be used, but from the viewpoints of safety, corrosiveness, and operation, iron (III) chloride is preferable. The amount of use is about 0.5 to 2.0 parts by weight, based upon 100 parts by weight of the vinyl ether.

For example, the reaction temperature is about -30°C to about 50°C and the reaction time is 1 to 20 hours.

In the present invention, the desired compound is synthesized by causing a nucleophile to act on the produced tetraalkoxypropane. As the above desired compound, 2-aminopyrimidine, 1-carboxylamidinopyrazole, etc. may be mentioned. As the tetraalkoxypropanes of the synthesis material, a symmetric form, asymmetric form, and their mixtures may be used.

### EXAMPLES

Examples will now be given to explain the present invention in more detail, but the present invention is by no means limited to these Examples.

### Example 1

A 300 ml four-necked flask equipped with a thermometer and stirrer was charged with 106.1 g (1.0 mol) of trimethyl orthoformate. While stirring, 0.3 g (0.002 mol) of anhydrous iron (III) chloride was added. While holding -15°C, 81.8 g (0.95 mol) of isopropyl vinyl ether was added over 5 hours, then the solution was aged for 2 hours. The weight percentage of the reaction solution at this stage was 178.7 g. The concentration of the desired 1,1,3,3-tetramethoxy-propane was 7.1% (production rate 7.7%).

### Example 2

A 300 ml four-necked flask equipped with a thermometer and stirrer was charged with 106.1 g (1.0 mol) of trimethyl orthoformate. While stirring, 0.3 g (0.002 mol) of anhydrous iron (III) chloride was added. While holding 2°C, 81.8 g (0.95 mol) of isopropyl vinyl ether was added over 5 hours, then the solution was aged at this temperature for 2 hours. The weight percentage of the reaction solution at this stage was 180.0 g. The concentration of the desired 1,1,3,3-tetra-methoxypropane was 9.0% (production rate 9.9%).

### Example 3

A 300 ml four-necked flask equipped with a thermometer and stirrer was charged with 106.1 g (1.0 mol) of trimethyl orthoformate. While stirring, 0.3 g (0.002 mol) of anhydrous iron (III) chloride was added. While holding 35°C, 81.8 g (0.95 mol) of isopropyl vinyl ether was added over 5 hours, then the mixture was aged at this temperature for 2 hours. The weight percentage of the reaction solution at this stage was 177.6 g. The concentration of the desired 1,1,3,3-tetramethoxypropane was 3.6% (production rate 3.9%).

### Example 4

A 2000 ml four-necked flask equipped with a thermometer and stirrer was charged with 541.4 g of trimethyl orthoformate (purity 98%, 5.00 mol). While stirring, 1.6 g (0.01 mol) of anhydrous iron (III) chloride was added. While maintaining -5 to 0°C, 413.3 g of n-propyl vinyl ether (purity 99%, 4.75 mol) was added over about 5 hours, then the mixture was allowed to stand overnight at room temperature while being stirred. Thereafter, the reaction solution was charged with 10.6 g of sodium carbonate, suction filtered, then distilled in vacuo to obtain 621.0 g of a mixture including 1,3,3-trimethoxy-1-(n-propoxy)propane in a concentration of 60.2% and 1,1,3,3-tetramethoxypropane in a concentration 32.5% (yield from n-propyl vinyl ether: 66.8%).

### Example 5

A 2000 ml four-necked flask equipped with a thermometer and stirrer was charged with 541.4 g of trimethyl orthoformate (purity 98%, 5.00 mol). While stirring, 1.6 g (0.01 mol) of anhydrous iron (III) chloride was added. While maintaining -5 to 0°C, 413.3 g of isopropyl vinyl ether (purity 99%, 4.75 mol) was added over about 5 hours, then the mixture was allowed to stand overnight at room temperature while being stirred. Thereafter, the reaction solution was charged with 10.6 g of sodium carbonate, suction filtered, then distilled in vacuo to obtain 607.5 g of a mixture of 1,3,3-trimethoxy-1-(isopropoxy)propane in a concentration of 62.9% and 1,1,3,3-tetramethoxy-propane in a concentration of 23.5% (yield from isopropyl vinyl ether: 70.0%).

### Example 6

A 2000 ml four-necked flask equipped with a thermometer and stirrer was charged with 541.4 g of trimethyl orthoformate (purity 98%, 5.00 mol). While stirring, 1.6 g (0.01 mol) of anhydrous iron (III) chloride was added. While maintaining -5 to 0°C, 480.5 g of n-butyl vinyl ether (purity 99%, 4.75 mol) was added over about 5 hours. The mixture was allowed to stand overnight at room temperature while being stirred. Thereafter, the reaction solution was charged with 10.6 g of sodium carbonate, suction filtered, then distilled in vacuo to obtain 564.3 g of a mixture containing 1,3,3-trimethoxy-1-(n-butoxy)propane in a concentration of 62.1% and 1,1,3,3-tetramethoxypropane in a concentration of 29.5% (yield from n-butyl vinyl ether: 57.1%).

### Example 7

A 2000 ml four-necked flask equipped with a thermometer and stirrer was charged with 541.4 g of trimethyl orthoformate (purity 98%, 5.00 mol). While stirring, 1.6 g (0.01 mol) of anhydrous iron (III) chloride was added. While maintaining -5 to 0°C, 485.5 g of isobutyl vinyl ether (purity 98%, 4.75 mol) was added over about 5 hours. The mixture was allowed to stand overnight at room temperature while being stirred. Thereafter, the reaction solution was charged with 10.6 g of sodium carbonate, suction filtered, then distilled in vacuo to obtain 746.3 g of a mixture of 1,3,3-trimethoxy-1-(isobutoxy)propane in a concentration of 58.2% and 1,1,3,3-tetramethoxypropane in a concentration of 24.9% (yield from isobutyl vinyl ether: 68.2%).

### Example 8

A 300 ml four-necked flask equipped with a thermometer and stirrer was charged with 148.1 g (1.0 mol) of triethyl orthoformate. While stirring, 0.3 g (0.002 mol) of anhydrous iron (III) chloride was added. While holding at 0°C, 81.5 g (0.95 mol) of n-propyl vinyl ether was added over 5 hours, then the mixture was aged as is for 1 hour. Thereafter, the reaction solution was distilled in vacuo to obtain 165.2 g of 1,3,3-triethoxy-1-(n-propoxy)propane in a purity of 39% (yield 28%) (114°C, 6.7 x 10⁻⁴ Mpa).

### Example 9

A 300 ml four-necked flask equipped with a thermometer and stirrer was charged with 148.1 g (1.0 mol) of triethyl orthoformate. While stirring, 0.3 g (0.002 mol) of anhydrous iron (III) chloride was added. While holding this at -30°C, 81.5 g (0.95 mol) of isopropyl vinyl ether was added over 5 hours, then the mixture was aged as is for 1 hour. Thereafter, the reaction solution was distilled in vacuo to obtain 1,3,3-triethoxy-1-(isopropoxy)propane of a purity of 73% in an amount of 133.2 g (yield 42%) and 1,1,3,3-tetraethoxypropane in a yield of 6.5% (111°C, 6.7 x 10⁻⁴ Mpa).

### Example 10

A 300 ml four-necked flask equipped with a thermometer and stirrer was charged with 148.1 g (1.0 mol) of triethyl orthoformate. While stirring, 0.3 g (0.002 mol) of anhydrous iron (III) chloride was added. While holding at 0°C, 81.5 g (0.95 mol) of isopropyl vinyl ether was added over 5 hours, then the mixture was aged as is for 1 hour. Thereafter, the reaction solution was distilled in vacuo to obtain 135.1 g of 1,3,3-triethoxy-1-(isopropoxy)propane of a purity of 47% (yield 27%) and 1,1,3,3-tetraethoxy-propane in a yield of 13.6% (111°C, 6.7 x 10⁻⁴ Mpa).

### Example 11

A 300 ml four-necked flask equipped with a thermometer and stirrer was charged with 106.1 g (1.0 mol) of trimethyl orthoformate. While stirring, 0.3 g (0.002 mol) of anhydrous iron (III) chloride was added. While holding at 20 to 32°C, 106.1 g (0.95 mol) of sec-butyl vinyl ether was added over 5 hours, then the mixture was raised in temperature to 40°C and aged for 2 hours. Thereafter, the reaction solution was distilled in vacuo to obtain 21.8 g of 1,3,3-trimethoxy-1-(sec-butoxy)propane of a purity of 35% (yield 3.9%) (79°C, 6.7 x 10⁻⁴ Mpa).

### Example 12: Synthesis of 2-aminopyrimidine

A 200 ml four-necked flask equipped with a thermometer, Dimroth condenser, and stirrer was charged with 20.8 g of concentrated hydrochloric acid (concentration 35.6%, 0.20 mol) and 23.0 g of guanidine hydrochlorate (purity 99.9%, 0.24 mol) while stirring. Next, 39.2 g of the mixture of Example 4 (purity 92.6%, 0.20 mol) was gradually added, then the inside of the tank was heated to 65°C and the stirring continued for 3 hours. Thereafter, while holding the solution temperature at 15°C, 39.4 g of an aqueous sodium hydroxide solution having a concentration of 32% was added. The production rate of 2-aminopyrimidine in the reaction mixture according to HPLC analysis was 72.6%.

### Example 13: Synthesis of 2-aminopyrimidine

A 200 ml four-necked flask equipped with a thermometer, Dimroth condenser, and stirrer was charged with 20.8 g of concentrated hydrochloric acid (concentration 35.6%, 0.20 mol) and 23.0 g of guanidine hydrochlorate (purity 99.9%, 0.24 mol) while stirring. Next, 42.4 g of the mixture of Example 5 (purity 86.4%, 0.20 mol) was gradually added, then the inside of the tank was heated to 65°C and the stirring continued for 3 hours. Thereafter, while holding the solution temperature at 15°C, 38.9 g of aqueous sodium hydroxide solution having a concentration of 32% was added. The production rate of 2-aminopyrimidine in the reaction mixture according to HPLC analysis was 72.8%.

### Example 14: Synthesis of 2-aminopyrimidine

A 200 ml four-necked flask equipped with a thermometer, Dimroth condenser, and stirrer was charged with 20.8 g of concentrated hydrochloric acid (concentration 35.6%, 0.20 mol) and 23.0 g of guanidine hydrochlorate (purity 99.9%, 0.24 mol) while stirring. Next, 41.5 g of the mixture of Example 6 (purity 91.6%, 0.20 mol) was gradually added, then the inside of the tank was heated to 65°C and the stirring continued for 3 hours. Thereafter, while holding the solution temperature at 15°C, 40.3 g of an aqueous sodium hydroxide solution having a concentration of 32% was added. The production rate of 2-aminopyrimidine in the reaction mixture according to HPLC analysis was 73.3%.

### Example 15: Synthesis of 2-aminopyrimidine

A 200 ml four-necked flask equipped with a thermometer, Dimroth condenser, and stirrer was charged with 20.8 g of concentrated hydrochloric acid (concentration 35.6%, 0.20 mol) and 23.0 g of guanidine hydrochlorate (purity 99.9%, 0.24 mol) while stirring. Next, 46.0 g of the mixture of Example 7 (purity 83.2%, 0.20 mol) was gradually added, then the inside of the tank was heated to 65°C and the stirring continued for 3 hours. Thereafter, while holding the solution temperature at 15°C, 40.4 g of an aqueous sodium hydroxide solution having a concentration of 32% was added. The production rate of 2-aminopyrimidine in the reaction mixture according to HPLC analysis was 73.6%.

### Example 16: Synthesis of 1-carboxylamidino-pyrazole

A 200 ml four-necked flask equipped with a thermometer, Dimroth condenser, and stirrer was charged with 50.0 g of distilled water and 27.3 g of aminoguanidine bicarbonate (purity 99.8%, 0.20 mol) while stirring, then 41.0 g of concentrated hydrochloric acid (concentration 35.6%, 0.40 mol) was added over 30 minutes. Next, the inside of the tank was heated to 80°C, 39.2 g of the mixture of Example 4 (purity 92.6%, 0.20 mol) was added over 2 hours and 50 minutes, then the stirring was continued for a further 1 hour and 30 minutes (rate of production of desired substance in reaction solution according to HPLC analysis: 96.1%). Next, this was cooled over ice and allowed to stand overnight, then the precipitated light yellow solid was obtained by filtration to obtain a needle-shaped wet solid in an amount of 22.6 g (yield 63.4%).

### Example 17: Synthesis of 1-carboxylamidino-pyrazole

A 200 ml four-necked flask equipped with a thermometer, Dimroth condenser, and stirrer was charged with 50.0 g of distilled water and 27.3 g of aminoguanidine bicarbonate (purity 99.8%, 0.20 mol) while stirring, then 41.0 g of concentrated hydrochloric acid (concentration 35.6%, 0.40 mol) was added over 30 minutes. Next, the inside of the tank was heated to 80°C, 42.6 g of the mixture of Example 5 (purity 86.4%, 0.20 mol) was added over 2 hours and 50 minutes, then the stirring was continued for a further 2 hours (rate of production of the desired substance in reaction solution according to HPLC analysis: 92.6%). Next, this was cooled over ice and allowed to stand overnight, then the precipitated yellow solid was obtained by filtration to obtain a needle-shaped wet solid in an amount of 21.8 g (yield 64.7%).

### Example 18: Synthesis of 1-carboxylamidino-pyrazole

A 200 ml four-necked flask equipped with a thermometer, Dimroth condenser, and stirrer was charged with 50.0 g of distilled water and 27.3 g of aminoguanidine bicarbonate (purity 99.8%, 0.20 mol) while stirring, then 41.0 g of concentrated hydrochloric acid (concentration 35.6%, 0.40 mol) was added over 30 minutes. Next, the inside of the tank was heated to 80°C, 41.8 g of the mixture of Example 6 (purity 91.6%, 0.20 mol) was added over 2 hours and 30 minutes, then the stirring was continued for a further 2 hours (rate of production of the desired substance in reaction solution according to HPLC analysis: 95.1%). Next, this was cooled over ice and allowed to stand overnight, then the precipitated light yellow solid was obtained by filtration to obtain a needle-shaped wet solid in an amount of 23.0 g (yield 64.0%).

### Example 19: Synthesis of 1-carboxylamidino-pyrazole

A 200 ml four-necked flask equipped with a thermometer, Dimroth condenser, and stirrer was charged with 50.0 g of distilled water and 27.3 g of aminoguanidine bicarbonate (purity 99.8%, 0.20 mol) while stirring, then 41.0 g of concentrated hydrochloric acid (concentration 35.6%, 0.40 mol) was added over 30 minutes. Next, the inside of the tank was heated to 80°C, 46.2 g of the mixture of Example 7 (purity 83.2%, 0.20 mol) was added over 2 hours and 30 minutes, then the stirring was further continued for 2 hours (rate of production of the desired substance in reaction solution according to HPLC analysis: 93.6%). Next, this was cooled over ice and allowed to stand overnight, then the precipitated light yellow solid was obtained by filtration to obtain 21.0 g of a needle shaped wet solid (yield 60.7%).

### Example 20 (Comparative Example): Synthesis of 2-aminopyrimidine

A 200 ml four-necked flask equipped with a thermometer, Dimroth condenser, and stirrer was charged with 20.8 g of concentrated hydrochloric acid (concentration 35.6%, 0.20 mol) and 23.0 g of guanidine hydrochlorate (purity 99.9%, 0.24 mol) while stirring. Next, 33.5 g of 1,1,3,3-tetramethoxypropane (purity 98.0%, 0.20 mol) was gradually added, then the inside of the tank was heated to 65°C and the stirring continued for 3 hours. Thereafter, while holding the solution temperature at 15°C, 38.9 g of an aqueous sodium hydroxide solution having a concentration of 32% was added. The production rate of 2-aminopyrimidine in the reaction mixture according to HPLC analysis was 79.4%.

### Example 21 (Comparative Example): Synthesis of 1-carboxylamidinopyrazole

A 300 ml four-necked flask equipped with a thermometer, Dimroth condenser, and stirrer was charged with 75.0 g of distilled water and 40.9 g of aminoguanidine bicarbonate (purity 99.8%, 0.30 mol) while stirring, then 61.6 g of concentrated hydrochloric acid (concentration 35.6%, 0.60 mol) was added over 45 minutes. Next, then inside of the tank was heated to 80°C, 50.2 g of 1,1,3,3-tetramethoxypropane (purity 98%, 0.30 mol) was added over 3 hours and 30 minutes, then stirring was further continued for 10 minutes (rate of production of the desired substance in reaction solution according to HPLC analysis: 97.3%). Next, this was cooled over ice and allowed to stand overnight, then the precipitated light yellow solid was obtained by filtration to obtain a needle-shaped wet solid in an amount of 37.0 g (yield 71.5%).

The results of analysis of the substances are described below.
1) 1,3,3-trimethoxy-1-(n-propoxy)propane
¹H-NMR (CDCl₃, TMS, 200 MHz)
δ(ppm); 0.94(t, J=14.9 Hz, 3H, CH3-CH2-CH2-O-), 1.62(m, 2H, CH3-CH2-CH2-O-), 1.91(m, 2H, >CH-CH2-CH<), 3.34(s, 9H, CH3-O-), 3.42(m, 2H, CH3-CH2-CH2-O-), 4.52(m, 2H, >CH-CH2-CH<)
¹³C-NMR (CDCl₃, TMS, 200 MHz)
δ(ppm); 10.6(CH3-CH2-CH2-O-), 23.0(CH3-CH2-CH2-O-), 36.6(>CH-CH2-CH<), 52.7(CH3-O-), 52.8(2C, CH3-O-), 67.9(CH3-CH2-CH2-O-), 101.0(CH3-O-CH(OCH3)-CH2-CH<), 101.8(CH3-0-CH(OCH3)-CH2-CH<)

| IR Analysis | | |
|---|---|---|
| Specific absorption band/cm⁻¹ | Derivation | Intensity |
| 2939 | C-H (anti-symmetric stretching) | vs |
| 1116 | C-O (stretching vibration) | vs |
| 1386 | C-H (in-plane variable angle vibration) | m |
| 905 | Fingerprint region | w |

2) 1,3,3-trimethoxy-1-(iso-propoxy)propane
¹H-NMR (CDCl₃, TMS, 200 MHz)
δ(ppm); 1.15, 1.22 (d, J=6.0 Hz, 6H, CH3-CH(CH3)-O-), 1.91(m, 1H, >CH-CH2-CH<), 3.31(s, 3H, CH3-O-), 3.33(s, 3H, CH3-O-), 3.34(s, 3H, CH3-O-), 3.86(m, 1H, CH3-CH(CH3)-O-), 4.62(t, J=11.7, 1H, CH3-O-CH(OCH3)-CH2-CH<), 4.49(t, J=12.1, 1H, CH3-O-CH(OCH3)-CH2-CH<)
¹³C-NMR (CDCl₃, TMS, 200MHz)
δ(ppm) ; 22.1, 23.1 (CH3-CH(CH3)-O-), 37.0(>CH-CH2-CH<), 51.6, 52.7, 53.0(CH3-O-), 69.1(CH3-CH(CH3)-O-), 99.2(CH3-O-CH(OCH3)-CH2-CH<), 101.9(CH3-O-CH(OCH3)-CH2-CH<)

| IR Analysis | | |
|---|---|---|
| Specific absorption band/cm⁻¹ | Derivation | Intensity |
| 2974 | C-H (anti-symmetric stretching) | vs |
| 1113 | C-0 (stretching vibration) | vs |
| 1383 | C-H (in-plane variable-angle vibration) | m |
| 905 | Fingerprint region | w |

3) 1,3,3-triethoxy-1-(n-propoxy)propane
¹H-NMR (CDCl₃, TMS, 200 MHz)
δ(ppm); 0.93(t, J=14.9 Hz, 3H, CH3-CH2-CH2-O-), 1.20(t, J=14.3Hz, 9H, CH3-CH2-O-), 1.61(m, 2H, CH3-CH2-CH2-O-), 1.96(m, 2H, >CH-CH2-CH<), 3.54(m, 6H, CH3-CH2-O-), 3.66(m, 2H, CH3-CH2-CH2-O-), 4.62(m, 2H, >CH-CH2-CH<)
¹³C-NMR (CDCl₃, TMS, 200 MHz)
δ(ppm); 10.7 (CH3-CH2-CH2-O-), 15.4 (CH3-CH2-O-), 23.2 (CH3-CH2-CH2-O-), 38.2 (>CH-CH2-CH<), 61.3 (CH3-CH2-O-), 67.6 (CH3-CH2-CH2-O-), 100.3 (>CH-CH2-CH<)

| IR Analysis | | |
|---|---|---|
| Specific absorption band/cm⁻¹ | Derivation | Intensity |
| 2976 | C-H (anti-symmetric stretching) | vs |
| 1116 | C-O (stretching vibration) | vs |
| 1376 | C-H (in-plane variable angle vibration) | m |
| 590 | Fingerprint region | w |

4) 1,3,3-triethoxy-1-(isopropoxy)propane
¹H-NMR (CDCl₃, TMS, 200 MHz)
δ(ppm) ; 1.16, 1.22(m, 9H, CH3-CH2-O-), 1.20(m, 6H, CH3-CH(CH3)-O-), 1.93(m, 1H, >CH-CH2-CH<), 3.51(m, 2H, CH3-CH2-O-), 3.58(m, 4H, CH3-CH2-O-), 3.87(m, 1H, CH3-CH(CH3)-O-), 4.64(t, J=11.7Hz, 2H, >CH-CH2-CH<)
¹³C-NMR (CDCl₃, TMS, 200 MHz)
δ(ppm); 15.4(CH3-CH2-O-), 22.3, 23.3(CH3-CH(CH3)-O-), 38.9(CH3-CH(CH3)-O-), 60.2, 61.2, 61.5(CH3-CH2-O-), 68.7(CH3-CH(CH3)-O-), 98.8(CH3-CH2-O-CH(OCH2CH3)-CH2-CH<), 100.4(CH3-CH2-O-CH(OCH2CH3)-CH2-CH<)

| IR Analysis | | |
|---|---|---|
| Specific absorption band/cm⁻¹ | Derivation | Intensity |
| 2975 | C-H (anti-symmetric stretching) | vs |
| 1114 | C-O (stretching vibration) | vs |
| 1380 | C-H (in-plane variable angle vibration) | m |
| 592 | Fingerprint region | w |

5) 1,3,3-trimethoxy-1-(sec-butoxy)propane
¹H-NMR (CDCl₃, TMS, 200 MHz)
δ(ppm); 0.91(m, 3H, CH3-CH2-CH(CH3)-O-), 1.11(d,J=6.34Hz,3H,CH3-CH2-CH(CH3)-O-), 1.20(m, 2H, CH3-CH2-CH(CH3)-0-), 1.52(m, 1H, CH3-CH2-CH(CH3)-O-), 1.92(m, 2H, >CH-CH2-CH<), 3.31(s,3H, CH3-O-), 3.33(s,3H, CH3-O-), 3.34(s,3H, CH3-O-), 4.5(m, 1H, CH3-O-CH(OCH3)-CH2-CH<), 4.6(m, 1H,CH3-O-CH(OCH3)-CH<)
^{13C}-NMR (CDCl₃, TMS, 200 MHz)
δ(ppm); 10.1,9.6(CH3-CH2-CH(CH3)-O-), 19.4, 20.4(CH3-CH2-CH(CH3)-O-), 29.3,30.1(CH3-CH2-CH(CH3)-O-), 36.1, 37.1(>CH-CH2-CH<), 51.8(CH3-O-), 52.8(CH3-O-), 53.1(CH3-O-), 73.9,74.9(CH3-CH2-CH(CH3)-O-), 98.9(CH3-O-CH(OCH3)-CH2-CH<), 100.2,101.8(CH3-O-CH(OCH3)-CH2-CH<)

| IR Analysis | | |
|---|---|---|
| Specific absorption band/cm⁻¹ | Derivation | Intensity |
| 2955 | C-H (anti-symmetric stretching) | vs |
| 1117 | C-O (stretching vibration) | vs |
| 1387 | C-H (in-plane variable angle vibration) | m |
| 907 | Fingerprint region | w |

### INDUSTRIAL APPLICABILITY

According to the present invention, it is possible to use the industrially useable propoxyvinyl ether to produce on an industrial scale 1,1,3,3-tetraalkoxypropane having a high reactivity and useful as a skeleton-forming agent used as a material for intermediates of pharmaceuticals and agrochemicals such as pyrazole and pyrimidine.

## Claims

1. A method for producing 1,1,3,3-tetraalkoxypropane having the formula (I): 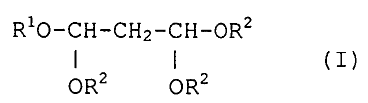 wherein R¹ indicates CH₃, C₂H₅ or C₃H₇, R² independently indicates CH₃ or C₂H₅, **characterized by** using, as starting materials, an orthoformic acid ester having the formula (II): 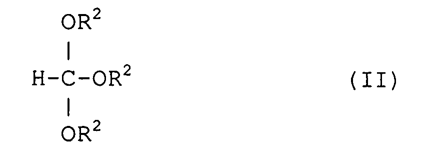 wherein R² is as defined above and
a vinyl ether having the formula (III):
CH₂=CH-OR³ (III)
wherein R³ indicates C₃H₇.

2. A method for producing a tetraalkoxy-propane as claimed in claim 1, wherein said tetraalkoxypropane is tetramethoxypropane, tetraethoxypropane, trimethoxy-mono(n- or iso-propoxy)propane and triethoxy-mono(n- or iso-propoxy)propane.

3. A method for producing a tetraalkoxypropane derivative, using, as a starting material, a tetraalkoxypropane having the formula (IV): 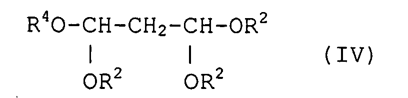 wherein R⁴ indicates CH₃, C₂H₅, C₃H₇ or C₄H₉, and R² independently indicates CH₃ or C₂H₅.

4. A method for producing a tetraalkoxypropane derivative as claimed in claim 3, wherein the tetraalkoxypropane, which is the starting material for the synthesis of said tetraalkoxypropane derivative, is tetramethoxypropane, tetraethoxypropane, trimethoxymono(propoxy or butoxy)propane, triethoxymono(propoxy or butoxy)propane or a mixture of the same.

5. A production method as claimed in claim 3, wherein the tetraalkoxypropane derivative is a pyrimidine derivative or pyrazole derivative.

6. A production method as claimed in claim 3 or 5, wherein the pyrimidine derivative is 2-aminopyrimidine.

7. A production method as claimed in claim 3 or 5, wherein the pyrazole derivative is 1-carboxylamidinopyrazole.
